# EUROPEAN PATENT APPLICATION

(11) **EP 0 631 764 A1**
(43) Date of publication of application: **04.01.1995**
(21) Application number: 94201775.7
(22) Date of filing: 22.06.1994
(51) Int. Cl.: A61F 2/30, A61F 2/38

(54) **Tibial part of a knee joint endoprosthesis**

(30) Priority: 22.06.1993 DE 4320520
(71) Applicant: ESKA Implants GmbH & Co., 23556 Lübeck (DE)
(72) Inventor: Schug, Martin, D-2355 Lubeck (DE); Thomas, Wolfram, I-00135 Rome (IT); Scholz, Jorg, D-14139 Berlin (DE); Henssge, Ernst-Joachim, D-23562 Lübeck (DE)
(74) Representative: Fuchs, Luderschmidt & Partner Patentanwälte

(57) **Abstract**

The tibial part of a knee joint endoprosthesis comprises a tibial plateau (4) made of metal, a plastic insert (6) on the tibial plateau and an anchoring element (8) on the lower side of the tibial plateau.

In order to increase the stability of the anchorage in the tibial bone, the anchoring element is designed in such a way that the diameter (d) of the largest circle inscribed in the horizontal cross section of the anchoring element directly beneath the tibial plateau is at least half the radius (R) of the largest circle inscribed on the tibial plateau.

## Description

This invention concerns the tibial part of a knee joint endoprosthesis, comprising a tibial plateau made of metal, a plastic insert on the tibial plateau and an anchoring element on the lower side of the tibial plateau.

Such a tibial part is known from German Patent 3,433,264 and is used at the proximal end of the human tibial bone that has first been resected appropriately. It serves as the support for a corresponding femoral part of a knee joint endoprosthesis that is implanted at the distal end of the human femur bone. With this known design, a slender anchoring shaft element is arranged on the underside of the tibial plateau (facing the bone) and is composed of two parts. This anchoring element is provided in order to secure the position and anchoring of the tibial part. The lower side of the tibial plateau and the anchoring elements have an open-celled or open-pored surface structure that allows the adjacent bony tissue to grow into the pores or cells that are accessible from the surface. This bony ingrowth gives an additional anchoring effect for the tibial part beneath the tibial plateau in the adjacent bony tissue.

Since the tibial part rests on the resected tibial bone forces acting vertically such as the weight of the body are transmitted to the adjacent bony tissue, especially the corticalis. It is necessary that care be taken to assure that transverse forces acting in the knee will also be absorbed well in order to assure that the tibial part will be secured adequately. In this regard, a disadvantage of this known embodiment has proven to be the very small horizontal projected area of the anchoring part, especially directly beneath the tibial plateau, which can absorb the transverse forces only to an inadequate extent.

The object of this invention is to improve the tibial part of the type described initially such that an improved anchoring is achieved, especially in order to effectively absorb transverse forces.

This problem is solved according to this invention due to the fact that the cross-sectional area of a first section of the anchoring element has a radial extent directly beneath the tibial plateau such that the diameter d of the largest circle that can be described on the tibial plateau corresponds at least to half the radius R of the largest circle that can be described on the tibial plateau.

The advantages of this invention include especially the fact that a large surface area is achieved due to the large radial extent of the anchoring element immediately below the tibial plateau. Transverse forces acting on the tibial part act against a comparatively large area of the adjacent bony tissue, thus resulting in a uniform distribution of the load over a large area into the adjacent bony tissue, and thus a reduction in local load peaks and therefore an improvement in the anchoring beneath the tibial plateau. Since the transverse forces acting on the tibial part directly beneath the tibial plateau are already acting on a comparatively large area of the adjacent bony tissue, the unfavorable lever effects with narrow shaft shaped anchoring elements are also avoided. In individual cases this type of anchoring may even make it unnecessary to use a shaft or it can be further improved by the use of an additional shaft in the area near the plateau.

The cross-sectional area of the first section of the anchoring element directly beneath the tibial plateau preferably has a radial extent such that the diameter d of the largest circle described corresponds at least to the radius of the largest circle described on the tibial plateau or is preferably just slightly smaller than two times the radius R of the largest circle described on the tibial plateau. Preferably the axial longitudinal extent of the first section of the anchoring element at right angles to the tibial plateau is smaller than the diameter d. Therefore the anchoring element has a ball-shaped thickened area directly beneath the tibial plateau. This thickened area of the first section of the anchoring element further improves the anchoring of the tibial part in the tibial bone.

It is especially preferable for the lower side of the tibial plateau and the anchoring element to have an open-celled or open-pored surface structure in at least some areas, making it possible for the adjacent spongy bony tissue to grow into the cells or pores that are accessible from the surface. This results in a strong bond between the bone and the implant.

The first section of the anchoring element is preferably designed so it has rotational symmetry, especially in the form of a spherical section, an ellipsoidal section, a hyperbolic section or a conical section that fills out the hollow space in the tibial bone radially to the extent that there is a remaining fixed distance from the corticalis. Due to the dynamically balanced or rotationally symmetrical design, forces acting horizontally on the tibial plateau are transmitted equally in all directions through the anchoring element over a large area and are transmitted uniformly to the adjacent bony tissue.

According to another embodiment of this invention, the first section of the anchoring element develops into a second section that is in the shape of a shaft that tapers distally and may also have an open-celled or open-pored surface structure over all or part of the surface. The second section is anchored in the cavity in the tibial bone in a known manner.

According to another preferred embodiment of this invention, the first section is flattened on the lower end and has a base surface that is parallel to the tibial plateau so that a separate second shaft section of suitable length and suitable diameter can be detachably anchored on the first section. Preferably a conical plug connection is provided as the detachable anchoring element between the two sections, whereby the first section, for example, has a conical recess facing the tibial plateau and the second section has a corresponding conical projection on its upper end that can be inserted with a locking effect into the conical recess. As an alternative, the conical projection on the first section can also be provided so that it can be inserted into a corresponding conical recess in the second section. In this way, the optimum shaft for the respective patient in terms of shape, size and surface structure can be selected and implanted during surgery.

According to one preferred embodiment of the invention, the radial extension of the anchoring element has wings, orientated radially towards the outer periphery of the plateau, directly beneath the tibial plateau, and their radial reach decreases as their axial distance from the tibial plateau increases. The radial wings preferably taper off towards their outer edge. In one preffered embodiment of the invention, two radial wings are moulded in a lateral-medial direction onto the anchoring element. Transverse forces exerted on the tibial part of this embodiment act on the enlarged surface area of the wing against the adjacent bony tissue such that a uniform distribution of load into the adjacent bony tissue is achieved over a large surface area. In this manner localised peak loads are avoided.

Advantageous embodiments of this invention are characterized by the features in the subclaims.

This invention is explained in greater detail below with reference to the figures which show the following:
Figure 1 shows a cross section through a first embodiment of a tibial part according to this invention;
Figure 2 shows a cross section through the tibial part from Figure 1 along line II and at right angles to the sectional plane from Figure 1;
Figure 3 shows a rear view of the tibial part of a second embodiment of the invention;
Figure 4 shows a side view of the embodiment of Figure 3; and
Figure 5 shows a plan view of the embodiment of Figure 4.

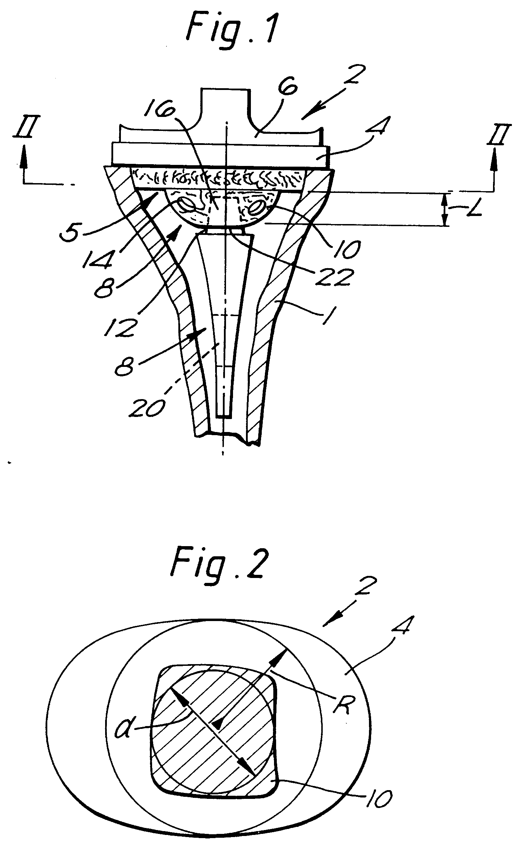

Figure 1 shows a tibial part 2 where the tibial plateau 4 made of metal and having a plastic insert 6 on which the condyles of the femur part of a knee joint endoprosthesis are supported. The tibial plateau 4 rests along its periphery on the resection edge of tibial bone 1.

A first section 10 of the anchoring element being hemispherical in shape and having a base surface 12 that is parallel to tibial plateau 4 on its lower end is provided on the lower side 5 of tibial bone 4. In the first section 10 there is a conical bore 16 facing the plateau. A second section in the form of shaft 20 is detachably anchored in the first section 10 of the anchoring element. Shaft 20 has a conical projection 22 facing upward on its upper enlarged end so the projection can be inserted into conical bore 16 of the first section 10 and locked there. The shape of shaft 20 approximates the shape of the cavity and it is tapered toward the bottom accordingly.

Several boreholes (not shown in the figure) running obliquely downward and outward toward the corticalis of the tibial bone 1 are provided through tibial plateau 4 and the first section 10 in order to also be able to anchor the tibial part with bone screws in the tibial bone if desired. Such an additional means of anchoring greatly increases the primary stability of the implant after implantation. Then the implant has an adequate anchoring effect in the bone immediately after surgery, thus allowing the bony tissue to grow into the open cells or pores of the surface structure.

In order to improve the anchoring of the tibial part in the tibial bone, an open-celled or open-pored surface structure 14 of metal into which the adjacent spongy bony tissue can grow is provided on the lower side 5 of tibial plateau 4 and on the first section 10 of the anchoring part. The radius of the first section 10 of the anchoring element is such that it has a sufficient distance from the bone wall of the tibial bone 1 at the side so that spongy tissue can surround the base of the shaft on all sides and can grow into the pores or cells of the surface structure 14 in the desired manner.

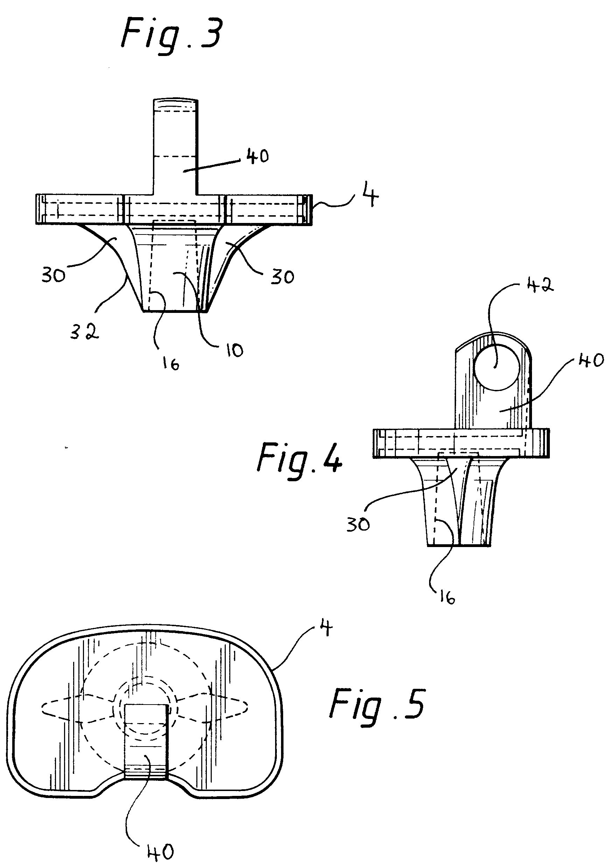

Figure 2 shows a cross section through tibial part 2 from Figure 1 along line II and at right angles to the cross-sectional plane of Figure 1. The cross-sectional area of the first section 10 of the anchoring element 8 which is shown with shading and is directly beneath the tibial plateau has a radial extent such that the diameter d of the largest circle described on the tibial plateau 4 is larger than the radius R of the largest circle described on tibial plateau 4. The axial length or longitudinal extent L of the first section 10 of the anchoring element 8 at right angles to the tibial plateau is such that L < d, preferably L < d/2. This yields a compact round shape for the first section 10 instead of the long thin shape conventionally known for such shafts.

Figures 3 to 5 concern a second embodiment of the invention in which for clarity the plastic insert on the tibial plateau and also the shaft found under the tibial plateau have been omitted. In order that the tibial part may be better anchored, the first section (10) of the anchoring element underneath the tibial plateau (4) has wings (30) oriented radially towards the outside, which - in the embodiment shown - are moulded in a lateral medial direction onto the anchoring element (8) and taper off towards their outer edge. The wings can be provided with an open-celled or open-pored surface structure - as can the entire underside of the tibial plateau. A projection (40) is moulded in a ventral-dorsal direction onto the tibial plateau and has a borehole (42) for receiving a transverse stabilising axle.

## Claims

1. Tibial part of a knee joint endoprosthesis with a tibial plateau made of metal, a plastic insert on the tibial plateau and an anchoring element on the lower side of the tibial plateau, characterized in that the cross-sectional area of the first section (10) of the anchoring element (8) directly beneath the tibial plateau (4) has a radial extent such that the diameter d of the largest circle described on the tibial plateau (4) corresponds to at least half the radius R of the largest circle described on tibial plateau (4).

2. Tibial part according to Claim 1, characterized in that the cross-sectional area of the first section (10) of the anchoring element (8) directly beneath the tibial plateau (4) has a radial extent such that the diameter d of the largest circle described on the tibial plateau corresponds at least to the radius R of the largest circle described on the tibial plateau.

3. Tibial part according to Claims 1 or 2, characterized in that the cross-sectional area of the first section (10) of the anchoring element (8) directly beneath the tibial plateau has a radial extent such that the diameter d of the largest circle described on the tibial plateau is only slightly smaller than two times the radius R of the largest circle described on the tibial plateau.

4. Tibial part according to one of the preceding claims, characterized in that the first section (10) of the anchoring element (8) has an open-celled or open-pored surface structure (14).

5. Tibial part according to one of the preceding claims, characterized in that the lower side (5) of the tibial plateau (4) has an open-celled or open-pored surface structure (14) over at least some areas.

6. Tibial part according to one of the preceding claims, characterized in that the first section (10) of the anchoring element (8) is in the shape of a spherical or conical section.

7. Tibial part according to one of Claims 1 to 5, characterized in that the first section (10) of the anchoring element (8) is in the shape of an ellipsoidal section or a hyperbolic section.

8. Tibial part according to one of the preceding claims, characterized in that the anchoring element (8) beneath the first section (10) develops into a shaft section (20) with a distal taper.

9. Tibial part according to one of the preceding claims, characterized in that the first section (10) and the second section (20) of the anchoring element (8) are detachably connected.

10. Tibial part according to one of the preceding claims, characterized in that the second section (20) has an open-celled or open-pored surface structure over at least some areas.

11. Tibial part according to one of the preceding claims, characterized in that the second section is made of a bioabsorbable material.

12. Tibial part according to one of the preceding claims, characterized in that several boreholes run obliquely to the outside through the tibial plateau and the first section.

13. Tibial part according to one of the preceding claims, characterized in that the first section (10) of the anchoring element (8) has an axial length L at right angles to the tibial plateau (4) where the length L is smaller than the diameter d.

14. Tibial part of a knee joint endoprosthesis comprising a tibial plateau made of metal, a plastic insert on the tibial plateau and an anchoring element on the underside of the tibial plateau, characterized in that the cross-sectional area of the first section (10) of the anchoring element (8) directly beneath the tibial plateau (4) has a radial extent such that the diameter d of the largest circle described on the tibial plateau (4) corresponds to at least half the radius R of the largest circle described on tibial plateau (4) and in that the anchoring element (8) is provided with wings (30) that extend radially outwards directly underneath the tibial plateau (4).

15. Tibial part as claimed in claim 14 characterised in that the wings (30) are provided with a radial taper towards their outer edge (32).

16. Tibial part as claimed in claim 14 or 15 characterised in that the wings are moulded in a lateral-medial direction onto a first section (10) of the anchoring element (8).

17. Tibial part as claimed in any preceding claim characterised in that there is a projection (40) on the tibial plateau provided with a borehole (42) for receiving a transverse stabilising axle.
